# EUROPÄISCHE PATENTANMELDUNG

(11) **EP 2 397 072 A1**
(43) Veröffentlichungstag der Anmeldung: **21.12.2011**
(21) Anmeldenummer: 10166405.0
(22) Anmeldetag: 17.06.2010
(51) Int. Cl.: A61B 5/04, A61B 5/0408, A61B 5/0424, A61B 5/0478, A61B 5/0492, A61N 1/04, A41D 13/12, A61N 1/30

(54) **Befeuchtete Sensorkontakteinheit**

(71) Anmelder: EMPA Eidgenössische Materialprüfungs- und Forschungsanstalt, 8600 Dübendorf (CH)
(72) Erfinder: Weder, Markus, 9230 Flawil (CH)
(74) Vertreter: Schmauder & Partner AG Patent- & Markenanwälte VSP

(57) **Zusammenfassung**

Um bei einer Sensorkontakteinheit (2) für einen elektrischen Kontaktes zwischen der Haut (30) und einem elektrischen Messsensors herzustellen, wird vorgeschlagen, dass auf einerelektrisch leitende textile Schicht (4) ein flächiges Befeuchtungselement (10) angeordnet und mit einem Flüssigkeitsreservoir (26) verbunden wird. Das flächige Befeuchtungselement (10) weist eine Innenschicht (14), eine Aussenschicht (16) sowie einen dazwischen angeordneten Befeuchtungsbereich (18) auf. Die Innenschicht (14) ist aus einem wasserdampfdurchlässigen Material gebildet.

## Beschreibung

### Technisches Gebiet

Die Erfindung betrifft eine Sensorkontakteinheit zur Messung von elektrischen Signalen eines Körpers, insbesondere eines menschlichen Körpers. Weiterhin betrifft die Erfindung einen Brustgurt mit einer entsprechenden Sensorkontakteinheit, insbesondere zur Messung von EKG-Daten.

### Stand der Technik

Die Messung von elektrischen Körpersignalen wie z.B. EKG (Elektrokardiogramm), EEG (Elektro-Enzephalogramm), EMG (Elektromyogramm) sind weit verbreitete Messmethoden zur Ableitung von Körpersignalen. Bisher wird das EKG im medizinischen Bereich praktisch ausschliessliich mit den Ag/AgCl Gelelektroden gemessen, welche um den Brustbereich auf die Haut geklebt wurden. Diese Elektroden bilden quasi einen Standard und werden weltweit angewendet, wenn es um EKG Messungen im medizinischen Bereich geht. Der Nachteil ist dass diese Elektroden nur eine limitierte Zeit von bis zu ca. 24 h funktionieren. Danach trocknen sie aus und ergeben kein geeignetes Signal mehr. Es hat sich jedoch herausgestellt, dass auch Langzeitmessungen wesentlich sein können. Dabei ist es wichtig, auch über mehere Tage das EKG mittels spezieller Holter - tragbare Logger - erfassen zu können. Dazu sind neue trockene Elektroden auf dem Markt aufgetaucht, die für EKG Messungen geeignet sein sollen. Beispielsweise sind Stickelektroden und Sensor-Shirts bekannt. Solche Elektroden funktionieren bei einer körperlichen Aktivität gut und ergeben gute Signale, insbesondere wenn die Person schwitzt oder somit eine feuchte Haut hat, aber eben nur solange die Haut feucht ist. Sobald aber die Elektroden trocken sind ist keine Messung mehr möglich. Der gesamte medizinische Bereich wo keine feuchte, sondern eine trockene Haut vorliegt, kann mit diesen Elektroden langzeitig nicht gemessen werden. Gerade dieser Bereich wäre aber aus wissenschaftlicher und mediziniescher Sicht sehr interessant, z.B. die Ueberwachung von Herzrisikopatienten oder generell bei älteren Leuten wo normalerweise eine trockene Haut vorliegt.

Ein weiteres Problem bei trockenen Elektroden sind die Bewegungsartefakte und die relativ hohe Pressung der Elektrode auf die Haut. Bei trockener Haut ist das Bewegen noch viel kritischer, d.h. die geringste Bewegung der Arme oder Beine kann zu extrem hohen Artefakten führen und aus diesen ein vernünftiges EKG-Signal herauszufiltern ist extrem schwierig.

Im Zusammenhang mit der Ueberwachung des Menschen können mit solchen Systemen wo eine Langzeiterfassung des EKG möglich ist auch Schlafversuche durchgeführt werden wo über die HRV Chaostheorie die Tiefschlafphasen exakt erkannt werden können oder z.B. als Sensor für die Stresserkennung von Feuerwehrleuten oder Piloten, Lastwagenfahrer etc..

Als Beispiele für den Stand der Technik sein hier die EP-A-0 011 813 aufgeführt, bei der ein flexibles Kissen mit Chemikalie zur Abgabe an die Haut vorgeschlagen wird, wobei die Unterseite zur Haut eine permeable Membrane aufweisen soll. Nachteilig daran ist, dass die Befeuchtung von der Hautseite kommt und dass der Vorschlag eine Chemikalie zwingend erfordert. Weiterhin erscheint die EP 0 409 067 A2 als Hintergrundinformation interessant, bei der eine benetzbare Elektrode mit Flüssigkeitsspeicherndem Absorbermaterial vorgeschlagen wird, wobei ein Absorber mit Ionischer Flüssigkeit zwischen der Elektrode und der Haut vorgesehen ist. In der US 5 057 072 A wird eine Elektrode mit Reservoir mit einer Chemikalie bzw. ionischen Flüssigkeit vorgeschlagen, wobei die Flüssigkeit auf der Hautseite ist und sich dahinter die Elektrode befindet. Auch hier erscheint es nachteilig, dass die Befeuchtung von der Hautseite kommt.

### Darstellung der Erfindung

Aufgabe der Erfindung ist es demnach, eine verbesserte Sensorkontakteinheit vorzuschlagen, bei der die beschriebenen Nachteile vom Stand der Technik zumindest vermieden werden können.

Diese Aufgabe wird gelöst durch die Sensorkontakteinheit zum Herstellen und Aufrechterhalten eines elektrischen Kontaktes zwischen der Haut einer Person und einer elektrischen Einheit gemäss Anspruch 1. Die Sensorkontakteinheit ist besonders geeignet zur Messung von elektrischen Daten der Person und umfasst eine erste textile Schicht, in oder auf der elektrisch leitende Elemente, insbesondere Leiterfäden, vorzugsweise aus Metall oder leitfähigen Kunststoffgarnen, angeordnet sind, ein flächiges Befeuchtungselement, welches auf der textilen Schicht aufliegt, einen elektrischer Anschluss, der mit der textilen Schicht in elektrischem Kontakt steht, und ein Flüssigkeitsreservoir. Das flächige Befeuchtungselement umfasst zumindest eine Innenschicht, eine Aussenschicht sowie einen dazwischen angeordneten Befeuchtungsbereich. Die Innenschicht ist aus einem wasserdampfdurchlässigen Material gebildet und der Befeuchtungsbereich ist mit dem Flüssigkeitsreservoir zur Versorgung des Befeuchtungsbereichs in Verbindung bringbar. Die vorliegende Erfindung beruht auf einer definierten, dampfförmigen Abgabe von Feuchtigkeit an die Elektroden. Diese langsame und definierte Abgabe von Wasserdampf geschieht von hinten, also von der den Elektroden abgewandten Seite und wird mittels eines speziellen Befeuchtungselementes ermöglicht, welches mit einem flexiblen Tank gespeist wird. Dabei haben die Massnahmen der Erfindung zunächst einmal zur Folge, dass langzeitig und unabhängig von chemischen Zusätzen, die als Hilfsmittel allerdings nicht grundsätzlich ausgeschlossen sind, die Sensorkontakteinheit guten Kontakt für die elektrischen Personendaten bereit stellt.

Bevorzugte Ausgestaltungen der Erfindung sind in den abhängigen Ansprüchen definiert.

Die Innenschicht ist zwar wasserdampfdurchlässig ausgebildet, kann aber durchaus wasserdicht sein, um ein Auslaufen der Flüssigkeit zu verhindern.

Die Innenschicht kann auch hydrophob und der Befeuchtungsbereich hydrophil ausgebildet sein, so dass ein Saugeffekt durch den hydrophilen Befeuchtungsbereich des Befeuchtungselements erzielt wird.

Die Aussenschicht kann vorteilhafterweise wasserdicht ausgebildet und zudem auch wasserdampfundurchlässig sein.

Für die Innenschicht wird eine Dicke von 1 bis 50 µm angegeben, wobei vorzugsweise Dicken von 5 bis 15 µm zum Einsatz kommen werden.

Das Befeuchtungselement ist vorteilhafterweise an den Seiten wasserdicht ausgebildet, vorzugsweise durch Verschweissen der Innenschicht mit der Aussenschicht im Bereich ihres Aussenbereichs.

Besonders vorteilhaft ist die Sensorkontakteinheit, wenn der Befeuchtungsbereich des Befeuchtungselementes flächige, kanalartige Strukturen aufweist, die von einer Flüssigkeit aus dem Flüssigkeitsreservoirs durchströmbar sind. Zur Versorgung ist eine in der Innen- und oder der Aussenschicht angebrachte, flächige Entlüftungsmembrane vorteilhaft, welche mit den Kanälen in Verbindung steht. Alternativ dazu ist ein in der Innen- und oder der Aussenschicht angebrachtes, flächiges Entlüftungsventil. Die flächige Ausgestaltung der eingeprägten kanalartigen Struktur ist vorzugsweise als Spiralen oder mäanderförmige Strukturen ausgebildet. Die Dicken der kanalartigen Struktur können variieren, wobei vorzugsweise die zuführenden, dem Flüssigkeitsreservoir näherstehenden Kanäle etwas dicker als die abführenden, der Entlüftungsmembran näherstehenden Kanäle sind.

Das Flüssigkeitsreservoir umfasst bevorzugt einen flexiblen Tank, vorzugsweise aus z.B. Polyäthylen oder PVC.

Die Sensorkantakteinheit umfasst in einer bevorzugten Ausgestaltung ein textiles Trägermaterial, welches sich flächig an die Aussenschicht anschliesst.

Die Leitfähigkeit kann dadurch hergestellt werden, dass in oder auf der ersten textilen Schicht Leiterfäden ein- oder aufgestickt sind oder dass die erste textile Schicht leitfähige Strukturen, insbesondere gewobene oder gestrickte Strukturen aufweist.

Gemäss einem weiteren Aspekt der vorliegenden Erfindung ist ein Brustsensor mit einer Sensorkontakteinheit gemäss dem ersten Aspekt ausgestattet und umfasst einen Brustgurt. Das Flüssigkeitsreservoir ist dann mittels eines flexiblen Tanks ausgebildet und in den Gurt integriert, sodass durch die leichte Pressung des Gurts auf den Tank ein Druck ausgeübt werden kann, der die Befeuchtung des Befeuchtungsbereichs bewirkt oder unterstützt.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption keinen besonderen Ausnahmebedingungen, so dass die in dem jeweiligen Anwendungsgebiet bekannten Auswahlkriterien uneingeschränkt Anwendung finden können.

### Kurze Beschreibung der Zeichnungen

Ausführungsbeispiele der Erfindung werden nachfolgend anhand der Zeichnungen näher beschrieben, dabei zeigen:
- Fig. 1: einen Schichtaufbau einer Sensorkontakteinheit gemäss einem bevorzugten Ausführungsbeispiel der vorliegenden Erfindung;
- Fig. 2: ein Befeuchtungselement nach Figur 1 in Draufsicht, schematisch.

### Wege zur Ausführung der Erfindung

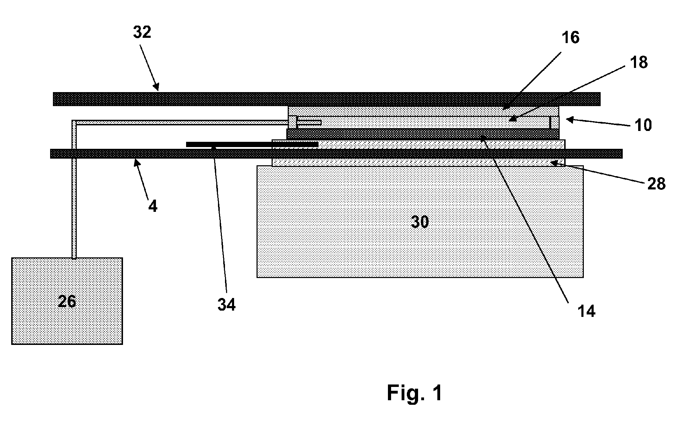

Die in Figur 1 in ihrer Schichtung dargestellte Sensorkontakteinheit 2 umfasst eine erste textile Schicht 4, in oder auf der elektrisch leitende Elemente, insbesondere Leiterfäden - in dem in Figur 1 gezeigten Ausführungsbeispiel als Stickelektroden 28 - angeordnet sind, ein flächiges Befeuchtungselement 10, welches auf der textilen Schicht 4 aufliegt, einen elektrischer Anschluss 34, der mit der ersten textilen Schicht 4 in elektrischem Kontakt steht, und ein Flüssigkeitsreservoir 26. Das flächige Befeuchtungselement 10 umfasst zumindest eine Innenschicht 14, eine Aussenschicht 16 sowie einen dazwischen angeordneten Befeuchtungsbereich 18. Die Innenschicht 14 ist im vorliegenden Ausführungsbeispiel aus einem wasserdampfdurchlässigen, aber wasserundurchlässigem Material gebildet und der Befeuchtungsbereich 18 steht mit dem Flüssigkeitsreservoir 26 zur Versorgung des Befeuchtungsbereichs mittels eines Zuführschlauches 24 in Verbindung.

Die Innenschicht ist zwar wasserdampfdurchlässig ausgebildet, kann aber durchaus wasserdicht sein, um ein Auslaufen der Flüssigkeit zu verhindern. Die Aussenschicht ist im vorliegenden Ausführungsbeispiel wasserdicht und zudem auch wasserdampfundurchlässig.

Die Innenschicht 14 hat im vorliegenden Ausführungsbeispiel eine Dicke von ca. 5 bis 15 µm.

Das Befeuchtungselement 10 ist vorteilhafterweise an den Seiten wasserdicht ausgebildet, vorzugsweise durch Verschweissen der Innenschicht mit der Aussenschicht 16 im Bereich ihres Aussenbereichs.

Im vorliegenden Ausführungsbeispiel weist der Befeuchtungsbereich 18 des Befeuchtungselementes 10 flächige, kanalartige Strukturen auf, die von der Flüssigkeit aus dem Flüssigkeitsreservoir 26 durchströmbar sind. Im Ausführungsbeispiel ist in der Aussenschicht eine flächige Entlüftungsmembrane 20 angebracht, welche mit den Kanälen in Verbindung steht. Alternativ dazu ist ein in der Innen- und oder der Aussenschicht angebrachtes, flächiges Entlüftungsventil. Die flächige Ausgestaltung der eingeprägten kanalartigen Struktur ist im vorliegenden Ausführungsbeispiel als Spiralen -alternativ als mäanderförmige Strukturen - ausgebildet. Die Dicken der kanalartigen Struktur variieren, wobei die zuführenden, dem Flüssigkeitsreservoir 26 näherstehenden Kanäle etwas dicker als die abführenden, der Entlüftungsmembran 20 näherstehenden Kanäle sind.

Das Flüssigkeitsreservoir 26 umfasst bevorzugt einen flexiblen Tank, im Ausführungsbeispiel aus Polyäthylen.

Die Sensorkontakteinheit 2 umfasst im Ausführungsbeispiel ein textiles Trägermaterial 32, welches sich flächig an die Aussenschicht 16 anschliesst. Im vorliegenden Ausführungsbeispiel bildet dieses textile Trägermaterial 32 einen Brustgurt aus. Das Flüssigkeitsreservoir 26 ist dabei mittels eines flexiblen Tanks ausgebildet und in den Gurt integriert, sodass durch die leichte Pressung des Gurts auf den Tank ein Druck ausgeübt werden kann, der die Befeuchtung des Befeuchtungsbereichs bewirkt oder zumindest unterstützt.

Die Leitfähigkeit ist - wie in Figur 1 dargestellt - dadurch bewirkt, dass in die erste textile Schicht 4 Leiterfäden zur Ausbildung einer Stickelektrode 28 eingestickt sind.

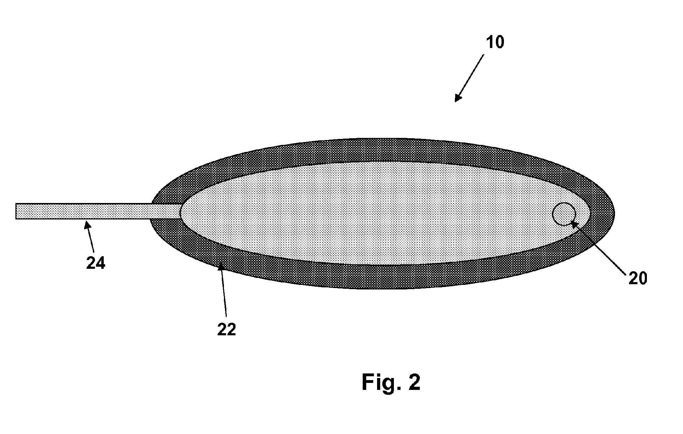

Durch die in Figur 2 dargestellte, praktisch luftdichte Randabschliessung des Befeuchtungselements 10 entsteht beim Verdunsten von Wasser ein Unterdruck, welcher bewirkt, dass Wasser vom Reservoir in das Befeuchtungselement gelangt. Es entsteht als ein Saugeffekt analog dem Blattspalt bei Baumblättern. Die Befüllung des Tanks mit destilliertem Wasser erfolgt im Ausführungsbeispiel mittels einer medizinischen Spritze und einem speziellen Verschluss mit Rückschlagventil in der Zuleitung von Tank zu Befeuchtungselementen.

Zur Verhinderung von Algen, Bakterien und Pilzen wird dem destilliertem Wasser noch ein Zusatz eines Bakterizids zugefügt.

### Bezugszeichenliste

- 2: Sensorkontakteinheit
- 4: erste textile Schicht
- 10: Befeuchtungselement
- 14: Innenschicht
- 16: Aussenschicht
- 18: Befeuchtungsbereich
- 20: Entlüftungsmembrane
- 22: Randverschweissung
- 24: Zuführschlauch
- 26: Flüssigkeitsreservoir
- 28: Stickelektrode
- 30: Haut
- 32: textiles Trägermaterial
- 34: elektrischer Anschluss

## Patentansprüche

1. Sensorkontakteinheit (2) zum Herstellen und Aufrechterhalten eines elektrischen Kontaktes zwischen der Haut (30) einer Person und einer elektrischen Einheit, insbesondere eines elektrischen Messsensors zur Messung von elektrischen Daten der Person, wobei die Sensorkontakteinheit umfasst
- eine erste textile Schicht (4), in oder auf der elektrisch leitende Elemente (28), insbesondere Leiterfäden, vorzugsweise aus Metall oder leitfähigen Kunststoffgarnen, angeordnet sind,
- ein flächiges Befeuchtungselement (10), welches auf der textilen Schicht (4) aufliegt,
- einen elektrischer Anschluss (34), der mit der ersten textilen Schicht (4) in elektrischem Kontrakt steht, und
- ein Flüssigkeitsreservoir (26),
**dadurch gekennzeichnet, dass** das flächige Befeuchtungselement (10) zumindest eine Innenschicht (14), eine Aussenschicht (16) sowie einen dazwischen angeordneten Befeuchtungsbereich (18) aufweist, wobei die Innenschicht (14) aus einem wasserdampfdurchlässigen Material gebildet ist und der Befeuchtungsbereich (18) mit dem Flüssigkeitsreservoir (26) zur Versorgung des Befeuchtungsbereichs (18) in Verbindung bringbar ist.

2. Sensorkontakteinheit nach Anspruch 1, **dadurch gekennzeichnet, dass** die Innenschicht (14) wasserdicht ausgebildet ist.

3. Sensorkontakteinheit nach Anspruch 1, **dadurch gekennzeichnet, dass** die Innenschicht (14) hydrophob und der Befeuchtungsbereich (18) hydrophil ausgebildet ist, so dass ein Saugeffekt durch den hydrophilen Befeuchtungsbereich (18) des Befeuchtungselements (10) erzielt wird.

4. Sensorkontakteinheit nach einem der Ansprüche 1 bis 3, **dadurch gekennzeichnet, dass** die Aussenschicht (16) wasserdicht, vorzugsweise auch wasserdampfundurchlässig, ausgebildet ist.

5. Sensorkontakteinheit nach einem der Ansprüche 1 bis 4, **dadurch gekennzeichnet, dass** die Innenschicht (14) eine Dicke von 1 bis 50 µm, vorzugsweise 5 bis 15 µm aufweist.

6. Sensorkontakteinheit nach einem der Ansprüche 1 bis 5, **dadurch gekennzeichnet, dass** das Befeuchtungselement (10) an dessen Seiten wasserdicht ausgebildet ist, vorzugsweise durch Verschweissen der Innenschicht (14) mit der Aussenschicht (16) im Bereich ihres Aussenbereichs (22).

7. Sensorkontakteinheit nach einem der Ansprüche 1 bis 6, **dadurch gekennzeichnet, dass** der Befeuchtungsbereich (18) des Befeuchtungselementes (10) flächige, kanalartige Strukturen aufweist, die von einer Flüssigkeit aus dem Flüssigkeitsreservoir (26) durchströmbar sind.

8. Sensorkontakteinheit nach Anspruch 7, **gekennzeichnet durch** eine in der Innen- und oder der Aussenschicht (14, 16) angebrachte, flächige Entlüftungsmembrane (20) oder ein flächiges Entlüftungsventil, welche jeweils mit den Kanälen in Verbindung steht.

9. Sensorkontakteinheit nach einem der Ansprüche 7 oder 8, **dadurch gekennzeichnet, dass** die flächige Ausgestaltung der eingeprägten kanalartigen Struktur, vorzugsweise als Spiralen oder mäanderförmige Strukturen, ausgebildet ist.

10. Sensorkontakteinheit nach einem der Ansprüche 7 bis 9, **dadurch gekennzeichnet, dass** die Dicken der kanalartigen Struktur variieren, wobei vorzugsweise die zuführenden, dem Flüssigkeitsreservoir näherstehenden Kanäle etwas dicker als die abführenden, der Entlüftungsmembran (20) bzw. des Entlüftungsventils näherstehenden Kanäle sind.

11. Sensorkontakteinheit nach einem der Ansprüche 1 bis 10, **dadurch gekennzeichnet, dass** das Flüssigkeitsreservoir (26) einen flexiblen Tank, vorzugsweise aus z.B. Polyäthylen oder PVC aufweist.

12. Sensorkontakteinheit nach einem der Ansprüche 1 bis 11, **gekennzeichnet durch** ein textiles Trägermaterial (32), welches sich flächig an die Aussenschicht anschliesst.

13. Sensorkontakteinheit nach einem der Ansprüche 1 bis 12, **dadurch gekennzeichnet, dass** in oder auf der ersten textilen Schicht (4) Leiterfäden (28) ein- oder aufgestickt sind.

14. Sensorkontakteinheit nach einem der Ansprüche 1 bis 12, **dadurch gekennzeichnet, dass** die erste textile Schicht (4) leitfähige Strukturen, insbesondere gewobene oder gestrickte Strukturen aufweist.

15. Brustsensor mit einer Sensorkontakteinheit nach einem der Ansprüche 1 bis 14 und einem Brustgurt, wobei das Flüssigkeitsreservoir (26) mittels eines flexiblen Tank ausgebildet ist und in den Gurt integriert ist, sodass durch die leichte Pressung des Gurts auf den Tank ein Druck ausgeübt werden kann, der die Befeuchtung des Befeuchtungsbereich (8) bewirkt oder unterstützt.
